Europäisches Patentamt

European Patent Office

Office européen des brevets

(19)

(11) Publication number: **0 073 251**
**B1**

(12) **EUROPEAN PATENT SPECIFICATION**

(45) Date of publication of patent specification: **11.06.86**

(51) Int. Cl.⁴: **A 61 K 37/64**

(21) Application number: **82900660.0**

(22) Date of filing: **01.03.82**

(86) International application number:
**PCT/JP82/00055**

(87) International publication number:
**WO 82/03011 16.09.82 Gazette 82/22**

(54) **AGENT FOR TREATING DISEASES OF RESPIRATORY ORGANS.**

(30) Priority: **02.03.81 JP 29650/81**

(43) Date of publication of application:
**09.03.83 Bulletin 83/10**

(45) Publication of the grant of the patent:
**11.06.86 Bulletin 86/24**

(84) Designated Contracting States:
**FR**

(56) References cited:
**GB-A-1 475 262**
**US-A-3 912 704**

**CHEMICAL ABSTRACTS, vol. 90, no. 25, June
18, 1979, page 223, abstract no. 199490w,
COLUMBUS OHIO (US), H. SUMI: "Low
molecular weight inhibitors produced by
protease digestion of human urinary trypsin
inhibitors"**

(73) Proprietor: **Mochida Pharmaceutical Co., Ltd.
1-7, Yotsuya Shinjuku-ku
Tokyo (JP)**

(72) Inventor: **OHNISHI, Haruo
4-14, Higashifunabashi 6-chome Funabashi-shi
Chiba 273 (JP)**
Inventor: **KOSUZUME, Hiroshi
1002-10, Honmokucho 4-chome Naka-ku,
Yokohama-shi
Kanagawa 231 (JP)**
Inventor: **SUZUKI, Yasuo
234-29, Ohaza-Sashima Kawaguchi-shi
Saitama 333 (JP)**
Inventor: **MOCHIDA, Ei
5-4, Komagome 2-chome Toshima-ku
Tokyo 170 (JP)**

(74) Representative: **Polus, Camille et al
c/o Cabinet Lavoix 2, Place d'Estienne d'Orves
F-75441 Paris Cedex 09 (FR)**

(56) References cited:
**CHEMICAL ABSTRACTS, vol. 76, no. 23, June 5,
1972, page 216, abstract no. 137665j,
COLUMBUS OHIO (US), G.J. PROKSCH et al.:
"Purification of the trypsin inhibitor from
human pregnancy urine"**

(56) References cited:
CHEMICAL ABSTRACTS, vol. 91, no. 9, August 27, 1979, page 263, abstract no. 70770d, COLUMBUS OHIO (US), H. SUMI et al.: "Purification of urinary trypsin-inhibitors by affinity chromatography and their modifications"

BIOLOGICAL ABSTRACTS, vol. 71, 1981, abstract no. 57060, PHILADELPHIA, PENNSYLVANIA (US), MURAMATU et al.: "Purification and characterization of urinary trypsin inhibitor, UTI68 from normal human urine, and its cleavage by human uropepsin"

## Description

Technical field

This invention relates to a therapeutic agent for respiratory diseases, and more specifically, to an agent for the treatment of respiratory diseases, comprising a urinary trypsin inhibitor and/or its decomposition product as the active ingredient.

Background art

Pulmonary emphysema characterized by the abnormal dilation of terminal bronchioles is frequently observed in respiratory diseases such as pneumonia, bronchial asthma, bronchitis, chronic bronchial stenosis, and bronchiectasis. Clinically, pulmonary emphysema is accompanied by such subjective symptoms as coughing, expectoration and dyspnea, and it is not uncommon that complications resulting from infections, etc., aggravates pulmonary emphysema to respiratory insufficiency or cor pulmonale.

Based on the fact that pulmonary emphysema is accompanied by serum $\alpha_1$-antitrypsin deficiency [Eriksson et al., J. Clin. Lab. Invest., 15, 132 (1963)], Eriksson advocated an autolysis theory for the induction of pulmonary emphysema in which he assumed that autolysis of the pulmonary tissues results from a decrease in $\alpha_1$-antitrypsin ($\alpha_1$AT), an inhibitor of protease which exists in local tissues or the blood stream, and these protease have their origin in leukocytes, alveolar macrophages, or microorganisms [Acta Medica Scandinavica, 177, 1 (1965)]. Martorana et al., however, reported that in an experimental pulmonary emphysema of hamsters induced by inhalation of papain, intravenous injection of $\alpha_1$AT resulted in a significant increase in the activity of the serum antitrypsin, but could not prevent pulmonary emphysema [Am. Respir. Dis., 118, 607 (1967)]. Many reports have also been made to the effect that much remains to be solved about the relation between serum $\alpha_1$AT and pulmonary emphysema because the serum $\alpha_1$AT of pulmonary emphysema patients does not always show a low level, and progesterone prevents pulmonary emphysema without causing an increase in $\alpha_1$AT activity [e.g., Susumu Harada et al., Internal Medicine, 32, 845 (1973)].

Human urinary trypsin inhibitor (hereinafter abbreviated as HUTI) is known to inhibit not only trypsin but also a wide range of tissue-damaging enzymes, and its urinary excretion increases in urine of patients with chronic inflammatory diseases such as infection or cancer.

GB—A—1 475 262 discloses injectable preparations containing human urinary trypsin inhibitor (HUTI) and their use as anti-inflammatory drug at a dose from 100 to 1000 µg unit/kg/day.

Further various urinary trypsin inhibitors are known.

Thus CA 91:70770d (1979) discloses two human urinary trypsin inhibitors with molecular weights of 67,000 and 22,500.

Biological Abstracts vol. 71 57060 (1981) discloses human urine trypsin inhibitors with molecular weights of 68,000, 25,000 and 30,000.

CA vol. 90 199490w (1979) discloses two different decomposition products of HUTI. These products inhibit chymotrypsin and plasmin activities as well as trypsin.

US—A—3 912 704 discloses of a protease inhibitor isolated from horse urine which inhibits proteolytic enzymes such as trypsin, chymotrypsin or plasmin and which differs from protease inhibitors isolated from human urine.

Disclosure of the invention

The present inventors conceived that, if pulmonary emphysema is, as asserted by Eriksson, due to autolysis caused by tissue-damaging enzymes which are activated in the inflamed site, HUTI may be involved in pathogenesis and/or progression of pulmonary emphysema and HUTI may be effective in the prophylaxis and treatment of pulmonary emphysema.

Furthermore, the present inventors have previously discovered that HUTI possesses anti-influenza virus activity and completed an invention using pharmaceutical agents which contain HUTI as the active ingredient.

Accordingly, HUTI is expected to be useful for the treatment of various respiratory diseases since it is effective not only in the direct treatment of pulmonary emphysema but also in the treatment of bacterial or viral infection of respiratory organs which may cause pulmonary emphysema.

The present inventors made investigations on the effect of HUTI on various respiratory diseases, and found, as shown in the experimental examples to be given hereinbelow, that HUTI exhibits an excellent effect against pulmonary emphysema, pneumonia and pulmonary fibrosis. HUTI is a glycoprotein having a molecular weight of 17,000 to 70,000.

UTI derived from an animal other than humans cannot practically be given to humans because it has antigenicity against humans. The present inventors had previously discovered that decomposition of UTI with protease yields a peptide which has an activity for inhibiting trypsin and an anti-influenza activity and does not have antigenicity against humans. Thus, the present inventors also questioned whether this UTI decomposition product has an effect similar to UTI. It was found that the decomposition product of HUTI has an effect equivalent to, or even greater than HUTI, against pneumonia, pulmonary emphysema and pulmonary fibrosis. These discoveries have led to this invention.

Thus an object of the present invention is a therapeutic agent for respiratory diseases, comprising a pharmaceutically effective amount of a human urinary trypsin inhibitor (HUTI), a decomposition product of urinary trypsin inhibitor or a mixture of these as active ingredient, wherein said human urinary trypsin inhibitor is an acid glycoprotein which has a molecular weight of about 67,000, an isoelectric point of pH 2—3 and contains 5—12% of carbohydrate, and said decomposition product of urinary trypsin inhibitor has a molecular weight of 6,000—9,000, an isoelectric point of pH 8.5—10, a maximum absorption at 278 nm, a nitrogen content of 15—17%, gives a positive nindyrin test, and is readily soluble in water and insoluble in ether, chloroform and ethanol, with the proviso that when the active ingredient is HUTI, then the therapeutic agent is not in an injectable form.

Human UTI has a molecular weight ranging from 17,000 to 70,000 and shows an activity for inhibiting trypsin [Proksh, J. Lab. Clin. Med., *79*, 491 (1972)], and it can be obtained, for example, by the method of Sumi et al. [J. Biochem., *83*, 141 (1978)].

Specifically, human urine is concentrated and passed through a column of arginine-Sepharose®. The column is then eluted with 2% aqueous ammonia containing 0.2M sodium chloride. Then, the eluate is subjected to a gel chromatography, using conventional methods, on a Sephadex® (TM) G-200 column to obtain a fraction of trypsin inhibitor. The urinary trypsin inhibitor so purified is an acid glycoprotein having a molecular weight of about 67,000 and an isoelectric point of pH 2 to 3 and containing 5 to 12% carbohydrates.

The decomposition product of UTI can be obtained by decomposing UTI with protease and then purifying the product by suitable, conventional purification methods used in biochemistry such as ion-exchange chromatography and gel filtration.

UTI of any mammalian origin can be used as the raw material for the UTI decomposition product, although UTI of human origin is especially preferred.

Decomposition of UTI with protease is carried out usually by generally known methods for decomposing proteins. For example, a solution of protease such as papain is added to UTI dissolved in a suitable buffer, and reacted at about 37°C for 10 to 60 minutes. The product is then purified by gel filtration, ion-exchange chromatography, or etc. to obtain a fraction having a molecular weight of 6,000 to 9,000. Preferably, before this purification, the reaction mixture is concentrated for instance by lyophilization, in order to achieve increased efficiency in the purification. The resulting UTI decomposition product can further be treated with another protease such as pepsin, to obtain a fraction having greater activity.

Various proteases, such as papain, pepsin, trypsin and α-chymotrypsin can be used as the protease for decomposing UTI. Papain and pepsin are especially preferred. These enzymes can be used not only in the form of a solution but also as a so-called insolubilised enzyme.

The decomposition product of UTI so obtained has a molecular weight of 6,000 to 9,000, an isoelectric point of pH 8.5 to 10, a maximum absorption at 278 nm, a nitrogen content of 15 to 17%, gives a positive ninhydrin test, and is readily soluble in water and insoluble in ether, chloroform and ethanol.

The effectiveness and toxicity of the HUTI and/or UTI decomposition product used in the invention are illustrated below by experimental examples.

Experimental Example 1

Suppressive effect on pulmonary emphysema (by intravenous administration):—

In accordance with the method of Martorana et al. [Can. J. Physiol. Pharmacol., *51*, 635 (1973)], HUTI (12 mg/kg), the decomposition product of UTI (1.2 mg/kg), and $α_1AT$ (300 mg/kg) were administered intravenously to male golden hamsters weighing about 70 g in groups each consisting of ten animals. Then, 70 ml of 3% papain solution was nebulized for 3 hours on the animals to permit inhalation and pulmonary emphysema was induced. One week later, the lungs were extirpated from the animals, and their specific static compliance (abbreviated SSC), specific lung volume at full inflation (abbreviated SVI), total deflation time (abbreviated TDT) and fragility were measured. SSC is expressed by

$$\frac{\Delta V}{5} \times W$$

wherein $\Delta V$ is the change in the lung volume which occurs when the pressure is decreased from 49 to 0 Pa (5 cmH$_2$O to 0 cmH$_2$O) after the air has been introduced into the lungs through the trachea, and W is the wet weight of the lungs. SVI is expressed by

$$\frac{P}{W} \text{ (ml/g)}$$

wherein P is the volume of the lungs upon introduction of air thereinto from the trachea at a pressure of 196.2 Pa (20 cmH$_2$O), and W is the wet weight of the lungs. TDT represents the time (in seconds) required for the lungs which have been inflated at a pressure of 196.2 Pa (20 cmH$_2$O) to shrink completely upon releasing the pressure. The fragility represents the ratio of damage of the lungs when they are inflated at a pressure of 196.2 Pa (20 cmH$_2$O). The results are shown in Table 1.

HUTI and the decomposition product of UTI showed significant suppressive effect on pulmonary emphysema as compared with α₁AT which showed no such activity.

TABLE 1

|  | SSC | SVI | TDT | Fragility (%) |
|---|---|---|---|---|
| Normal group | 0.45±0.02 | 6.38±0.29 | 6.55±0.23 | 0 |
| Control group | 0.96±0.07 | 11.00±0.57 | 9.53±0.34 | 50 |
| HUTI-administered group | 0.56±0.04** | 7.41±0.32** | 7.02±0.32** | 10 |
| UTI decomposition product-administered group | 0.57±0.04** | 7.11±0.44** | 7.27±0.31** | 10 |
| α₁AT-administered group | 0.89±0.10 | 10.86±0.63 | 9.60±0.13 | 50 |

** $P < 0.01$.

Experimental Example 2
  Suppressive effect on pulmonary emphysema (by inhalation):—
  Male golden hamsters weighing about 70 g were used in groups each consisting of ten animals. Animals were anesthetized with sodium pentobarbital and 1 mg of papain per 100 g of body weight was injected into the trachea of the animals. After they woke up from the anesthesia, 10 ml of HUTI preparation in a concentration of 20 mg/ml or the decomposition product of UTI in a concentration of 2 mg/ml was nebulized on the animals for 40 minutes to permit inhalation. The lungs were extirpated one week later, and their SSC, SVI, TDT and fragility were measured in the same way as in Experimental Example 1. The results are shown in Table 2.
  HUTI and the decomposition product of UTI showed significant suppressive effect on pulmonary emphysema.

TABLE 2

|  | SSC | SVI | TDT | Fragility (%) |
|---|---|---|---|---|
| Normal group | 0.48±0.04 | 5.88±0.38 | 6.32±0.25 | 0 |
| Control group | 0.82±0.04 | 9.30±0.27 | 8.34±0.20 | 40 |
| HUTI-administered group | 0.55±0.06** | 7.02±0.22** | 6.98±0.38** | 10 |
| UTI decomposition product-administered group | 0.58±0.07** | 7.12±0.26** | 6.77±0.34** | 10 |

** $P < 0.01$.

Experimental Example 3
  Suppressive effect on pneumonia (1):—
  A tube was inserted in the trachea of white Japanese male rabbits weighing 2.6±0.2 kg in groups each consisting of 7 animals, and 0.1N hydrochloric acid was introduced for 3 days to the trachea through the tube at a rate of 1 ml/kg/day to induce deglutition pneumonia. HUTI (12 mg/kg), the decomposition product of UTI (1.2 mg/kg) or a mixture comprising HUTI and the UTI decomposition product in a ratio of 10:1 (6.6 mg/kg) was administered to the animals with deglutition pneumonia for a total of 5 days (the administration was conducted before the introduction of HCl during the first 3 days when the introduction of HCl was continued). The number of animals which died within 10 days after the beginning of the experiment in each group was compared with that of the control group. The results are shown in Table 3.
  The mortality by deglutition pneumonia decreased by the administration of HUTI or the UTI decomposition product. Administration of a mixture of these also resulted in a decrease in mortality by deglutition pneumonia.

## TABLE 3

| | Number of dead animals |
|---|---|
| Control group | 6/7 |
| HUTI-administered group | 1/7* |
| UTI decomposition product-administered group | 2/7* |
| Mixture-administered group | 0/7** |

* $P < 0.05$,
** $P < 0.01$.

Experimental Example 4

Suppressive effect on pneumonia (2):—

Without anesthesia, 1 ml/kg/day of 0.1N hydrochloric acid was introduced through a Nelaton's catheter to the trachea of white Japanese male rabbits weighing 2.6±0.2 kg in groups each consisting of 7 animals. Starting on the next day, the animals were allowed to inhale 0.1N hydrochloric acid over a total period of 3 days, twice daily each for 10 minutes by using a nebulizer. Thus, deglutition pneumonia was induced. HUTI (12 mg/kg) or its decomposition product (1.2 mg/kg) was administered to the trachea of the animals with deglutition pneumonia during four days during which HCl was administered. The administration was conducted before the introduction of HCl. On the fifth day after the beginning of the experiment, the carotid arteries of the animals were cut to bleed them to death. The chest of each animal was opened and a large amount of physiological saline under a constant pressure was perfused through the pulmonary artery to remove the blood from the lungs. Then, the lungs were extirpated, and homogenized by adding 9 ml, per gram of the lung, of physiological saline. Phospholipid of the resulting homogenate was quantitated. The lung homogenate was centrifuged at 3,000 rpm for 10 minutes. The supernatant liquid was diluted with physiological saline. One milliliter of 95% ethanol was added to 1 ml of the diluted solution, and the mixture was stirred for 15 seconds. Fifteen minutes later, remaining small stable foam on the gas-liquid interface was observed as a measurement of the existence of a surface active substance in the lung. When the foam formed a ring about the wall of the test tube, the result was evaluated as "positive", and the level of the surface active substance was expressed as the maximum dilution ratio at which the "positive" result was obtained. The results are shown in Table 4.

HUTI and the decomposition product of UTI showed a significant suppressive activity on deglutition pneumonia.

## TABLE 4

| | Lung weight (g) | Pulmonary phospholipid (mg/g of lungs) | Foam stability (dilution ratio) |
|---|---|---|---|
| Normal group | 9.2±0.4 | 29.0±0.8 | 4.5±0.4 |
| Control group | 18.1±2.0 | 22.6±1.3 | 2.0±0.3 |
| HUTI-administered group | 11.7±1.3** | 26.8±1.1* | 4.0±0.3** |
| UTI decomposition product-administered group | 12.3±1.5* | 27.2±1.4* | 4.1±0.5** |

* $P < 0.05$,
** $P < 0.01$.

Experimental Example 5

Suppressive effect on pulmonary fibrosis:—

In accordance with the method of Imano [Lung and Heart, 21, 31 (1974)], bleomycin in a dose of 10 mg/kg was administered intraperitoneally for 10 days to dd-strain male mice weighing 20 g in groups each consisting of 10 animals. HUTI (12 mg/kg) or the decomposition product of UTI (1.2 mg/kg) was injected intravenously once a day for four weeks. At the end of that period, the amount of collagen was measured in accordance with the method of Hayashi ["*Kosankinbyo Kenkyu Zasshi*", *24*, 253 (1972)] using as a parameter the uptake of $^{14}$C-hydroxyproline into collagen in the lungs. The results are shown in Table 5. UTI and the decomposition product of UTI decreased the amount of collagen.

TABLE 5

| | Amount of collagen in the lungs ($^{14}$C-hydroxyproline d.p.m.) |
| --- | --- |
| Control group | 3800±430 |
| HUTI-administered group | 2200±3.0 |
| UTI decomposition product-administered group | 2450±450 |

Experimental Example 6

Acute toxicity:—

HUTI, the decomposition product of UTI and a 10:1 mixture of UTI and UTI decomposition product were dissolved in physiological saline and injected intravenously or intraperitoneally in a dose of 2 g/kg into ddy-strain mice weighing about 20 g in groups each consisting of 10 animals. The conditions of the animals were observed over a period of one week. Changes in body weight during the period of observation were observed to be the same as those of a group to which physiological saline alone was given, and no death of the animals were noted. Autopsy conducted after one week showed no abnormality in the animals.

It is evident from the above experimental examples that HUTI and the decomposition product of UTI remarkably suppress pulmonary emphysema, pneumonia and pulmonary fibrosis, and that the doses at which these activities are exhibited are safe enough in view of the results of the acute toxicity test. Accordingly, HUTI, the decomposition product of UTI, and a mixture of these are very useful drugs for the prophylaxis and treatment of various respiratory diseases including pulmonary emphysema, pneumonia and pulmonary fibrosis.

The best mode for carrying out the invention

The therapeutically effective amount of the HUTI and/or UTI decomposition product in accordance with this invention is 1 to 1,000 mg and preferably 50—500 mg per day, but can be optionally increased or decreased depending upon the condition of the patient or the route of administration.

Usually, the HUTI and UTI decomposition product in accordance with this invention are administered in the form of an injection or an inhalant. They may also be used in the form of an oral preparation or an intrarectal preparation. Suitably, the injection and inhalant are lyophilized preparations which are used after being dissolved in a suitable solvent. Suitable oral preparations are capsules, tablets, granules, powders and oral liquid preparations. In formulating HUTI or UTI decomposition product into oral preparations, it is favorable to seal these compounds in liposomes to improve absorption after oral administration. An intrarectal suppository is suitable as the intrarectal preparation. In formulating these preparations, conventional methods can be used with conventional pharmaceutically acceptable carriers, excipients, etc.

The procedure by which HUTI is isolated and purified will be explained below, using examples. These examples are, however, as a matter of course, not intended to limit the method to the ones explained in the examples, and methods generally used in isolation and purification may also be used.

Example 1

According to the method of Proksh [J. Lab. Clin. Med., *79*, 491 (1972)], 650 liters of urine, obtained from healthy humans, was concentrated, then dialyzed against deionized water overnight. The dialyzed urine was adjusted to pH 7.8 by adding 1N sodium hydroxide solution. Then the urine was passed through a DEAE-cellulose column (20×80 cm) equilibrated with 0.05M tris-HCl buffer solution (pH 7.8) so as to have UTI adsorbed on the column. Then the column was washed with 40 liters of the same buffer solution and eluted with the same buffer solution containing 0.3M of sodium chloride. The eluate was then heated at 60 degrees centigrade for 20 minutes to inactivate proteases, which may exist as an impurity, for prevention of the degradation of HUTI, and 16 grams of weight of crude HUTI was obtained. Crude HUTI, 16 grams of weight, was dissolved in 0.02M glycine-HCl buffer solution (pH 3.4) and dialyzed against distilled water overnight and applied to DEAE-cellulose column (8.0×60 cm) equilibrated with the same buffer solution. Then the column was washed with 10 liters of the same buffer solution and then with 10 liters of the same buffer solution containing 0.2M sodium chloride, and eluted with 8 liters of the same buffer solution containing 0.4M sodium chloride. Active fraction was concentrated by ultrafiltration and the concentrated eluate was applied to a Sephadex® G-100 (Pharmacia) gel filtration column (10×95 cm), which was

7

prepared so that it was free from pyrogens, using physiological saline as a developer. Purified HUTI thus obtained possessed a purity of 95 percent and a specific activity of 2500 TIU/mg.

Specific activity of HUTI was measured according to the method of Kassell [Methods in Enzymology, *19*, 844 (1970)] taking the amount of UTI which inhibited 1 microgram of bovine trypsin (3000 NFU/mg) by 100 percent as 1 TIU.

The formulation of the pharmaceutical composition in accordance with this invention is illustrated below by Examples without any intention of limiting it to these Examples.

Example 2
    Lyophilized injection:—
    Four grams of human UTI was dissolved in 200 ml of physiological saline, and the solution was filtered through a membrane filter to sterilize it. Ten milliliters of the filtrate were filled in each of a number of sterilized glass containers, and lyophilized. The containers were sealed up to obtain lyophilized injections.

Example 3
    Lyophilized inhalants:—
    Seven grams of papain was added to 2 g of human UTI dissolved in 150 ml of 0.015M phosphate buffer (pH 7.2). The mixture was left to stand at 37°C for 30 minutes, and then lyophilized. The lyophilized powder was dissolved in 52 ml of 0.154M sodium chloride solution. The solution was subjected to a gel filtration through a column (10×83 cm) of Sephadex® G-100 equilibrated with the same sodium chloride solution as above and a fraction having a molecular weight of 7,000 to 9,000 was collected. The protein amount of the resulting fractions was quantitated by the method of Lory [J. Biol. Chem., *139*, 265 (1951)] using bovine serum albumin as a standard protein. Physiological saline was added to the fractions to give a concentration of 10 mg/ml. The solution was filtered to sterilize through a membrane filter and 10 ml of the filtrate was filled in each of a number of glass containers and lyophilized. Then, the glass containers were sealed up to provide lyophilized inhalants.

Example 4
    Liquid injection:—
    Pepsin (20 mg) was added to 1 g of the fraction having a molecular weight of 7,000 to 9,000 obtained by the method of Example 3. The mixture was left to stand at 37°C for 10 minutes, and lyophilized after adjusting it to pH 8 with 4N sodium hydroxide solution. The lyophilized powder was dissolved in 50 ml of water. The solution was passed through a column (5×50 cm) of Sephadex® G-25 equilibrated with 0.02M phosphate buffer (pH 8.0) to replace the buffer, and then adsorbed on a column (2.6×15 cm) of CM-Sepharose® equilibrated with the same buffer as above. Then, the column was eluted by a linear concentration gradient method using the same buffer as above containing 0.2M sodium chloride, and a fraction having a molecular weight of 6,000 to 7,000 were collected. The amount of protein in this fraction was measured by the same method as in Example 3 and then the protein concentration of the solution was adjusted to 10 mg/ml by physiological saline. The solution was filtered through a membrane filter to sterilize it. Five milliliters of the filtrate was filled in each of a number of glass containers, and the containers were sealed up to obtain liquid injections.

Example 5
    Liquid injection:—
    One gram of the decomposition product of UTI having a molecular weight of 6,000 to 7,000 obtained by the method of Example 4 and 10 g of human UTI were dissolved in 1,100 ml of physiological saline. The solution was filtered through a membrane filter to sterilize it. Five milliliters of the solution was filled in each of a number of glass containers. The containers were sealed up to obtain liquid injections.

Example 6
    Tablets:—

| | |
|---|---|
| Human UTI | 4 g |
| Lactose | 3.2 g |
| Potato starch | 1.5 g |
| Polyvinyl alcohol | 0.15 g |
| Magnesium stearate | 0.15 g |

Each of the above ingredients were weighed. Human UTI, lactose and potato starch were uniformly mixed. An aqueous solution of polyvinyl alcohol was added to the mixture, and granules were prepared by a wet-type granulation method. The granules were dried, and mixed with magnesium stearate. The mixture was compressed and tabletted to obtain tablets each weighing 100 mg.

Example 7

Oral liquid preparation:—

Five grams of swine UTI [Carlsson et al., Enzyme, *18*, 176 (1974)] was dissolved in 400 ml of 0.015M phosphate buffer (pH 7.2), and 15 g of papain was added to the solution. The mixture was left to stand at 37°C for 30 minutes, and then lyophilized. The lyophilized powder was dissolved in 250 ml of 0.154M sodium chloride solution. The solution was subjected to a gel filtration through a column (14×90 cm) of Sephadex® G-100 equilibrated with the same solution of NaCl as above, and a fraction having a molecular weight of 7,000 to 9,000 was collected. The amount of protein in the resulting fraction was measured by the above-mentioned method of Lory et al., and the concentration of the fraction was adjusted to 100 mg/ml. After the solution was filtered through a membrane filter to sterilize it, simple syrup was added to obtain oral liquid preparations.

Example 8

Liposome-sealed preparation:—

Egg-yolk lecithin, cholesterol and diacetylphosphate were mixed at a molar ratio of 7:2:1 and 100 mg of the mixture was dissolved in 12.5 ml of chloroform. The chloroform solution was applied to inner wall of a glass flask to form a thin film. This film and 25 ml of phosphate buffer containing 100 mg of human UTI was mixed and a suspension was prepared. The suspension was sonicated and then centrifuged at 110,000 g. The precipitate was suspended in 3 ml of physiological saline and sterilized. Thus a liposome-sealed UTI preparation was obtained.

Industrial applicability

The pressent invention is useful as a therapeutic agent for respiratory diseases, such as pulmonary emphysema, pneumonia and pulmonary fibrosis.

**Claims**

1. A therapeutic agent for respiratory diseases, comprising a pharmaceutically effective amount of a human urinary trypsin inhibitor (HUTI), a decomposition product of urinary trypsin inhibitor or a mixture of these as active ingredient, wherein said human urinary trypsin inhibitor is an acid glycoprotein which has a molecular weight of about 67,000, an isoelectric point of pH 2—3 and contains 5—12% of carbohydrate, and said decomposition product of urinary trypsin inhibitor has a molecular weight of 6,000—9,000, an isoelectric point of pH 8.5—10, a maximum absorption at 278 nm, a nitrogen content of 15—17%, gives a positive ninhydrin test, and is readily soluble in water and insoluble in ether, chloroform and ethanol, with the proviso that when the active ingredient is HUTI, then the therapeutic agent is not in an injectable form.

2. A therapeutic agent for respiratory diseases, comprising a pharmaceutically effective amount of a decomposition product of urinary trypsin inhibitor, wherein said decomposition product of urinary trypsin inhibitor has a molecular weight of 6,000—9,000, an isoelectric point of pH 8.5—10, a maximum absorption at 278 nm, a nitrogen content of 15—17%, gives a positive ninhydrin test, and is readily soluble in water and insoluble in ether, chloroform and ethanol.

3. A therapeutic agent according to claim 2, wherein the decomposition product of urinary trypsin inhibitor is of human origin.

4. A therapeutic agent according to claim 2, wherein the decomposition product of urinary trypsin inhibitor is of an animal origin other than human.

5. A therapeutic agent according to any one of the claims 1 to 4, which contains the active ingredient together with liquid or solid vehicles and/or excipients, with the proviso that when the active ingredient is HUTI the liquid vehicles and/or the excipients are not suitable for injection.

6. A therapeutic agent according to any one of the claims 1 to 5, wherein the subject disease is one or more diseases selected from the pulmonary emphysema, pneumonia and pulmonary fibrosis.

7. A therapeutic agent according to any one of the claim 2 to claim 6, in the form of an injectable preparation.

8. A therapeutic agent according to any one of the claims 1—6, in the form of an oral preparation.

9. A therapeutic agent according to any one of the claims 1—6, in the form of an inhalant.

10. A therapeutic agent according to any one of the claims 1—6, in the form of an intrarectal suppository.

11. The use of a human urinary trypsin inhibitor, decomposition product of urinary trypsin inhibitor or a mixture of these, for the manufacture of a pharmaceutical composition for the treatment of respiratory diseases which comprises a pharmaceutically effective amount of a human urinary trypsin inhibitor, a decomposition product of urinary trypsin inhibitor or a mixture of these as active ingredient in association with a pharmaceutically acceptable carrier, wherein said human urinary trypsin inhibitor is an acid glycoprotein which has a molecular weight of about 67,000, an isoelectric point of pH 2—3 and contains 5—12% of carbohydrate and said decomposition product of urinary trypsin inhibitor has a molecular weight of 6,000—9,000, an isoelectric point of pH 8.5—10, a maximum absorption at 278 nm, a nitrogen content of 15—17%, gives a positive ninhydrin test, and is readily soluble in water and insoluble in ether, chloroform and ethanol.

**0 073 251**

12. The use according to claim 11, wherein the subject disease is one or more diseases selected from the pulmonary emphysema, pneumonia and pulmonary fibrosis.

**Patentansprüche**

1. Therapeutisches Mittel für Erkrankungen der Atmungswege, enthaltend eine pharmazeutisch wirksame Menge eines Trypsin-Inhibitors aus menschlichem Urin (HUTI), ein Zersetzungsprodukt eines urinalen Trypsin-Inhibitors oder eine Mischung aus diesen als Wirkstoff, worin der Trypsin-Inhibitor aus menschlichem Urin ein Säure-Glycoprotein ist, das ein Molekulargewicht von etwa 67 000 und einen isoelektrischen Punkt beim pH 2 bis 3 aufweist und 5 bis 12% Kohlenhydrat enthält, und das Zersetzungsprodukt eines urinalen Trypsin-Inhibitors ein Molekulargewicht von 6 000 bis 9 000, einen isoelektrischen Punkt beim pH 8,5 bis 10, ein Absorptionsmaximum bei 278 nm und einen Stickstoffgehalt von 15 bis 17% aufweist, einen positiven Ninhydrin-Rest liefert und in Wasser leicht löslich sowie in Ether, Chloroform und Ethanol unlöslich ist, mit der Maßgabe, daß dann, wenn der Wirkstoff HUTI ist, das therapeutische Mittel nicht in injizierbarer Form vorliegt.

2. Therapeutisches Mittel für Erkrankungen der Atmungswege, enthaltend eine pharmazeutisch wirksame Menge eines Zersetzungsprodukts eines urinalen Trypsin-Inhibitors, worin das Zersetzungsprodukt eines urinalen Trypsin-Inhibitors ein Molekulargewicht von 6 000 bis 9 000, einen isoelektrischen Punkt beim pH 8,5 bis 10, ein Absorptionsmaximum bei 278 nm und einen Stickstoffgehalt von 15 bis 17% aufweist, einen positiven Ninhydrin-Test, liefert und in Wasser leicht löslich sowie in Ether, Chloroform und Ethanol unlöslicht ist.

3. Therapeutisches Mittel nach Anspruch 2, worin das Zersetzungsprodukt eines urinalen Trypsin-Inhibitors humanen Urprungs ist.

4. Therapeutisches Mittel nach Anspruch 2, worin das Zersetzungsprodukt eines urinalen Trypsin-Inhibitors animalischen, nicht-humanen Ursprungs ist.

5. Therapeutisches Mittel nach irgendeinem der Ansprüche 1 bis 4, das den Wirkstoff zusammen mit flüssigen oder festen Trägern und/oder Streckmitteln enthält, mit der Maßgabe, daß dann, wenn der Wirkstoff HUTI ist, die flüssigen Träger und/oder Streckmittel nicht zur Injektion geeignet sind.

6. Therapeutisches Mittel nach irgendeinem der Ansprüche 1 bis 5, wobei die zu behandelnde Erkrankung eine oder mehrere Erkrankungen ausgewählt aus der aus Lungenemphysem, Lungenentzündung und Lungenfibrose sind.

7. Therapeutisches Mittel nach irgendeinem der Ansprüche 2 bis 6 in Form eines injizierbaren Präparats.

8. Therapeutisches Mittel nach irgendeinem der Ansprüche 1 bis 6 in Form eines oralen Präparats.

9. Therapeutisches Mittel nach irgendeinem der Ansprüche 1 bis 6 in Form eines Inhalationsmittels.

10. Therapeutisches Mittel nach irgendeinem der Ansprüche 1 bis 6 in Form eines intrarektalen Suppositoriums.

11. Verwendung eines Trypsin-Inhibitors aus menschlichem Urin, eines Zersetzungsprodukts eines urinalen Trypsin-Inhibitors oder einer Mischung aus diesen für die Herstellung einer pharmazeutischen Zusammensetzung zur Behandlung von Erkrankungen der Atmungswege, die eine pharmazeutisch wirksame Menge eines Trypsin-Inhibitors aus menschlichem Urin, ein Zersetzungsprodukt eines urinalen Trypsin-Inhibitors oder eine Mischung aus diesen als Wirkstoff in Verbindung mit einem pharmazeutisch geeigneten Träger enthält, wobei der Trypsin-Inhibtor aus menschlichem Urin ein Säure-Glycoprotein ist, das ein Molekulargewicht von etwa 67 000 und einen isoelektrischen Punkt beim pH 2 bis 3 aufweist und 5 bis 12% Kohlenhydrat enthält, und das Zersetzungsprodukt eines urinalen Trypsin-Inhibitors ein Molekulargewicht von 6 000 bis 9 000, einen isoelektrischen Punkt beim pH 8,5 bis 10, ein Absorptionsmaximum bei 278 nm und einen Stickstoffgehalt von 15 bis 17% aufweist, einen positiven Ninhydrin-Test liefert und in Wasser leicht löslich sowie in Ether, Chloroform und Ethanol unlöslich ist.

12. Verwendung nach Anspruch 11, wobei die zu behandelnde Erkrankung eine oder mehrere Erkrankungen ausgewählt aus der aus Lungenemphysem, Lungenentzündung und Lungenfibrose sind.

**Revendications**

1. Un agent thérapeutique pour le traitement des maladies respiratoires comprenant une quantité pharmaceutiquement efficace d'un inhibiteur urinaire humain de la trypsine (HUTI), un produit de décomposition d'un inhibiteur urinaire de la trypsine ou un mélange de ceux-ci comme ingrédient actif, dans lequel ledit inhibiteur urinaire humain de la trypsine est une glycoprotéine acide qui a un poids moléculaire d'environ 67 000, un point iso-électrique de pH 2—3 et contient 5—12% de glucides, et ledit produit de décomposition d'un inhibiteur urinaire de la trypsine a un poids moléculaire de 6 000 à 9 000, un point iso-électrique de pH 8,5—10, un maximum d'absorption à 278 nm, une teneur en azote de 15—17%, donne une réaction positive dans le test à la ninhydrine et est facilement soluble dans l'eau et insoluble dans l'éther, le chloroforme et l'éthanol, sous réserve que lorsque l'ingrédient actif est l'HUTI, l'agent thérapeutique n'est pas sous une forme injectable.

2. Un agent thérapeutique pour le traitement des maladies respiratoires comprenant une quantité pharmaceutiquement efficace d'un produit de décomposition d'un inhibiteur urinaire de la trypsine, dans lequel ledit produit de décomposition d'un inhibiteur urinaire de la trypsine a un poids moléculaire de 6 000 à 9 000, un point isoélectrique de pH 8,5—10, une absorption maximale à 278 nm, une teneur en azote de 15 à 17%, donne une réaction positive dans le test à la ninhydrine et est facilement soluble dans l'eau et insoluble dans l'éther, le chloroforme et l'éthanol.

3. Un agent thérapeutique selon la revendication 2 dans lequel le produit de décomposition de l'inhibiteur urinaire de la trypsine est d'origine humaine.

4. Un agent thérapeutique selon la revendication 2 dans lequel le produit de décomposition d'un inhibiteur urinaire de la trypsine provient d'un animal autre que l'homme.

5. Un agent thérapeutique selon l'une quelconque des revendications 1 à 4 contient l'ingrédient actif avec des véhicules et/ou excipients liquides ou solides, sous réserve que lorsque l'ingrédient actif est l'HUTI, les véhicules et/ou les excipients liquides ne conviennent pas à l'injection.

6. Un agent thérapeutique selon l'une quelconque des revendications 1 à 5 dans lequel la maladie en question est une ou plusieurs des maladies choisies parmi l'emphysème pulmonaire, la pneumonie et la fibrose pulmonaire.

7. Un agent thérapeutique selon l'une quelconque des revendications 2 à 6 sous forme d'une préparation injectable.

8. Un agent thérapeutique selon l'une quelconque des revendications 1 à 6 sous forme d'une préparation orale.

9. Un agent thérapeutique selon l'une quelconque des revendications 1 à 6 sous forme d'un produit pour inhalation.

10. Un agent thérapeutique selon l'une quelconque des revendications 1 à 6 sous forme d'un suppositoire rectal.

11. L'emploi d'un inhibiteur urinaire humain de la trypsine, d'un produit de décomposition d'un inhibiteur urinaire de la trypsine ou d'un mélange de ceux-ci pour la préparation d'une composition pharmaceutique pour le traitement des maladies respiratoires qui comprend une quantité pharmaceutiquement efficace d'un inhibiteur urinaire humain de la trypsine, d'un produit de décomposition d'un inhibiteur urinaire de la trypsine ou d'un mélange de ceux-ci comme ingrédient actif en association avec un véhicule pharmaceutiquement acceptable, dans laquelle ledit inhibiteur urinaire humain de la trypsine est une glycoprotéine acide qui a un poids moléculaire d'environ 67 000, un point isoélectrique de pH 2—3 et contient 5 à 12% de glucides, et ledit produit de décomposition d'un inhibiteur urinaire de la trypsine a un poids moléculaire de 6 000 à 9 000, un point iso-électrique de pH 8,5—10, une absorption maximale à 278 nm, une teneur en azote de 15 à 17%, donne une réaction positive dans le test à la ninhydrine et est facilement soluble dans l'eau et insoluble dans l'éther, le chloroforme et l'éthanol.

12. L'emploi selon la revendication 11 dans lequel la maladie en question est une ou plusieurs maladies chosies parmi l'emphysème pulmonaire, la pneumonie et la fibrose pulmonaire.